# EUROPEAN PATENT APPLICATION

(11) **EP 1 829 529 A1**
(43) Date of publication of application: **05.09.2007**
(21) Application number: 07002900.4
(22) Date of filing: 25.04.2003
(51) Int. Cl.: A61K 9/14, A61K 9/20, A61K 47/32, A61K 9/48, A61K 9/70, A61F 13/00, A61F 2/00

(54) **Controlled release compositions of estradiol metabolites**

(30) Priority: 02.05.2002 US 377490 P
(62) Divisional of application: 03724207.0
(71) Applicant: PR Pharmaceuticals, Inc., Fort Collins, CO 80524 (US)
(72) Inventor: Allison, Dean S., Fort Collins, CO 80526 (US); Schmidt, Paul G., Niwot, CO 80503 (US); Hudnut, Paul S., Fort Collins, CO 80525 (US)
(74) Representative: Thornton, Neil

(57) **Abstract**

The present invention provides improved sustained release formulations of estradiol metabolites, including 2-hydroxyestradiol, 2-methoxyestradiol, 4-hydroxyestradiol and 4-methoxyestradiol, useful for therapeutic treatments. The invention also provides methods of producing sustained release forms of estradiol metabolites. The compositions of the present invention include microparticles, nanoparticles, patches, crystals, gels, rods, stints, pellets, discs, lozenges, wafers, capsules, films, microcapsules, nanocapsules, hydrogels, liposomes, implants and vaginal rings. Compositions also include formulations for transdermal and intravenous delivery of estradiol metabolites. The present invention provides numerous improvements over previous forms of estradiol metabolites, such advantages including the sustained release of normally short half-life compounds to maintain therapeutic blood levels.

## Description

### Cross Reference to Related Applications

This application claims priority from United States Provisional Patent Application No. 60/377,490 filed May 3, 2002 entitled, "Estradiol Metabolites and Ester Derivatives of Estradiol Metabolites," which application is hereby incorporated by this reference in its entirety

### Field of the Invention

This invention relates generally to sustained release forms of estradiol metabolites, as well as methods of making and using them.

### Background of the Invention

Estradiol is converted into different derivatives through metabolic processes *in* v*ivo*. Two particular types of metabolites are the catecholestrogens and the methoxyestradiols. The catecholestrogens, 2-hydroxyestradiol and 4-hydroxyestradiol, are created by hydroxylation of estrogen via cytochrome P450 enzymes. The catecholestrogens can be methylated by catechol-O-methyl-transferase to create the methoxyestradiols, 2-methoxyestradiol and 4-methoxyestradiol.

Estradiol metabolites have been reported to have an effect on a number of cellular processes. They apparently inhibit angiogenesis and the polymerization and organization of tubulin in actively growing cells and induce apoptosis in some cells. In addition, 2-hydroxyestradiol and 2-methoxyestradiol appear to affect cholesterol levels in ovariectomized rats and to inhibit adipose cell proliferation in culture, while 2-hydroxyestradiol and 2-methoxyestradiol apparently decreases the effects of obesity, metabolic syndrome, and vascular and renal dysfunction in obese rats. Estradiol metabolites are also reported to be beneficial in the treatment of end-stage renal disease and asthma. Additionally, estradiol metabolites appear to be effective antifungal agents.

Beneficial effects of estradiol metabolites have also been reported for cancer treatment. 2-methoxyestradiol appears to decrease the growth of lung cancer cells in culture when administered with wild-type p53, to inhibit the growth of human pancreatic and prostate cancer cells and to be toxic to osteosarcoma cells. 2-methoxyestradiol was also reported to decrease the growth rate of neuroblastoma cells and tumors of the pituitary gland. Estradiol metabolites also apparently increase the intracellular accumulation of superoxide anions in rapidly dividing cells and enhance the effects of existing cancer treatments, such as radioimmunotherapy.

Thus, estradiol metabolites may be useful in the treatment or prevention of a variety of diseases. Unfortunately, naturally occurring estradiol metabolites have poor bioavailability and a short half life, and the beneficial effects appear to be tied to a prolonged period of treatment. A need exists for pharmaceutical formulations of estradiol metabolites, which increase the duration of action of the metabolites without necessitating frequent administrations, which would be undesirable in both animal and human patients. The development of a sustained release system for estradiol metabolites would provide an improved therapeutic option for treatment of a wide-variety of veterinary and human diseases.

### Summary of the Invention

In accordance with the purpose(s) of this invention, as embodied and broadly described herein, this invention, in one aspect, relates to sustained release formulations of estradiol metabolites and methods of making and using the same.

In certain embodiments, the compositions and methods may comprise an estradiol metabolite and a material providing for sustained release. Such material providing for sustained release may be selected from the group consisting of microparticles, nanoparticles, patches, crystals, gels, rods, stints, pellets, discs, lozenges, wafers, capsules, films, microcapsules, nanocapsules, hydrogels, liposomes, implants and vaginal rings. In addition, the invention further provides for hydrophilic polymers. In certain embodiments, the present invention provides compositions of matter or methods utilizing prodrugs of estradiol metabolites. Such prodrugs may be ester derivatives of estradiol metabolites. In other embodiments, the estradiol metabolite may be derivatized.

Hydrophilic polymers of use in the present invention may include, but are not limited to, poly(ethylene glycol), poly(propylene glycol) and copolymers of poly(ethylene glycol) and poly(propylene glycol).

In a particular embodiment, estradiol metabolites are catecholestrogens or methoxyestradiols. In particular embodiments, they are selected from the group consisting of 2-methoxy estradiol, 2-hydroxy estradiol, 4-methoxy estradiol and 4-hydroxy estradiol.

In other particular embodiments, microparticles or nanoparticles may comprise a biodegradable polymer selected from the group consisting ofpoly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, biodegradable polyurethanes, blends and copolymers thereof.

Estradiol metabolites of use in the present invention may be selected from the group consisting of 2-methoxy estradiol, 2-hydroxy estradiol, 4-methoxy estradiol and 4-hydroxy estradiol. In a certain embodiment, the ester derivative of an estradiol metabolite is selected from the group consisting of 3-benzoyl-2-methoxy estradiol; 17-benzoyl-2-methoxy estradiol; 17-acetyl-2-methoxy estradiol; 3-acetyl-2-methoxy estradiol; 3,17-dibenzoyl-2-methoxy estradiol; 3,17-diacetyl-2-methoxy estradiol; 3-benzoyl-4-methoxy estradiol;17-benzoyl-4-methoxy estradiol; 17-acetyl-4-methoxy estradiol; 3-acetyl-4-methoxy estradiol; 3,17-dibenzoyl-4-methoxy estradiol; 3,17-diacetyl-4-methoxy estradiol; 3-benzoyl -2-hydroxy estradiol;17-benzoyl-2-hydroxy estradiol; 17-acetyl-2-hydroxy estradiol; 3-acetyl-2-hydroxy estradiol; 3,17-dibenzoyl-2-hydroxy estradiol; 3,17-diacetyl-2-hydroxy estradiol; 2,3-dibenzoyl-2-hydroxy estradiol; 2,17-dibenzoyl-2-hydroxy estradiol; 2,17-diacetyl-2-hydroxy estradiol; 2,3-diacetyl-2-hydroxy estradiol; 2,3,17-tribenzoyl-2-hydroxy estradiol; 2,3,17-triacetyl-2-hydroxy estradiol; 3-benzoyl-4-hydroxy estradiol; 17-benzoyl-4-hydroxy estradiol; 17-acetyl-4-hydroxy estradiol; 3-acetyl-4-hydroxy estradiol; 3,17-dibenzoyl-4-hydroxy estradiol; 3,17-diacetyl-4-hydroxy estradiol; 3,4-dibenzoyl-4-hydroxy estradiol; 4,17-dibenzoyl-4-hydroxy estradiol; 4,17-diacetyl-4-hydroxy estradiol; 3,4-diacetyl-4-hydroxy estradiol; 3,4,17-tribenzoyl-4-hydroxy estradiol; 3,4,17-triacetyl-4-hydroxy estradiol.

Derivatives include but are not limited to dicarboxylic acid compounds, diacids, polar compounds and ionic compounds.

The compositions of the present invention may be applied transdermally, such as by buccal, oral, ocular, nasal, rectal or vaginal application. The compositions and methods of the present invention may also utilize estradiol metabolites in a eutectic mixture.

The compositions and methods of the present invention may utilize sustained release forms of estradiol metabolites produced by any method known in the art. In particular embodiments, such production methods will include but are not limited to, spray drying a solution of polymer and estradiol metabolite dissolved in an organic solvent; wet emulsification including a continuous and discontinuous phase followed by solvent removal; selective extraction of an oil phase solvent and emulsion. Any of the production methods may further provide an annealing step.

The compositions and methods of the present invention may be useful to treat an individual.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### Description of the Invention

The present invention may be understood more readily by reference to the following detailed description of particular embodiments of the invention and the Examples included therein.

Before the present compounds, compositions, and/or methods are disclosed and described, it is to be understood that this invention is not limited to specific synthetic methods, specific reagents or to laboratory or manufacturing techniques, as such may, of course, vary, unless it is otherwise indicated. It is also to be understood that the terminology used herein is for the purpose of describing particular embodiments only and is not intended to be limiting.

### Definitions

For the purposes of the present invention, the following terms shall have the following meanings:

For the purposes of the present invention, the term "biodegradable" refers to polymers that dissolve or degrade *in vivo* within a period of time that is acceptable in a particular therapeutic situation. This time is typically less than five years and usually less than one year after exposure to a physiological pH and temperature, such as a pH ranging from 6 to 9 and a temperature ranging from 25°C to 38°C.

The term "analog" and its cognates refer to any molecule that demonstrates estradiol metabolite activity. Such molecule may be a synthetic analog, fragment of estradiol metabolite or endogenous biological molecule other than an estradiol metabolite capable of estradiol metabolite-like activity. In sum, an estradiol metabolite analog refers to any molecule that demonstrates bioactivity similar or greater than an estradiol metabolite itself.

For the purposes of the present invention, the term "prodrug" refers to any modification of an estradiol metabolite, including a physical or chemical alteration that results in an increased plasma circulation time, increased encapsulation efficiency and/or increased water solubility. The chemical modification or modifications to the drug are reversible upon administration to an individual by endogenous mechanisms. The product of such endogenous mechanisms is an estradiol metabolite.

For the purposes of the present invention, the term "estradiol metabolite" includes any molecule that results from the metabolic breakdown of estradiol; and any molecule derived from an estradiol metabolite, such as a prodrug; or an analog.

For the purposes of the present invention, the term "drug" may refer to any estradiol metabolite, any estradiol metabolite analog, or estradiol metabolite prodrug.

Moreover, for the purposes of the present invention, the term "a" or "an" entity refers to one or more of that entity; for example, "a prodrug" or "an estradiol metabolite molecule" refers to one or more of those compounds or at least one compound. As such, the terms "a" or "an", "one or more" and "at least one" can be used interchangeably herein. It is also to be noted that the terms "comprising," "including," and "having" can be used interchangeably. Furthermore, a compound "selected from the group consisting of' refers to one or more of the compounds in the list that follows, including mixtures (i.e. combinations) of two or more of the compounds.

For the purpose of the present invention, a "eutectic" mixture is composed of two or more substances that melt at the lowest possible temperature.

According to the present invention, an isolated or biologically pure molecule is a compound that has been removed from its natural milieu. As such, "isolated" and "biologically pure" do not necessarily reflect the extent to which the compound has been purified. An isolated compound of the present invention can be obtained from its natural source, can be produced using laboratory synthetic techniques or can be produced by any such chemical synthetic route.

For the purposes of the present invention, ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

Finally, for the purposes of the present invention, the term "individual" means an animal or human of either gender.

Reference will now be made in detail to particular embodiments of the invention.

Naturally occurring estradiol metabolites have a short plasma half-life. Oral bioavailability is low, in part due to rapid hepatic metabolism. In addition, some indications that can be treated using estradiol metabolites, estradiol metabolite analogs, or estradiol metabolite prodrugs, such as diabetic nephropathy or obesity, require prolonged administration of the drug(s). Development of estradiol metabolites, estradiol metabolite analogs, or estradiol metabolite prodrugs dosage forms for delivery over extended time periods is a novel way to administer these particular therapeutics in a useful formulation.

Two main strategies exist to prolong the duration of exposure to rapidly metabolized drugs, particularly steroids. The first is to increase plasma circulation time by chemically modifying the steroid with organic acids to form steroid ester prodrugs. After delivery, the steroid ester bond is cleaved to form the parent compound by endogenous enzymes. Physical and chemical properties imparted to the steroid by the organic acid, or other modifying compound, govern the rate at which the parent compound is released from its prodrug form. In this way, the plasma circulation time can be increased in a controlled manner. Sustained exposure to a steroid ester prodrug may be realized by any delivery route, including intravenous, peroral, intramuscular, subcutaneous, transdermal, rectal, ocular, and so on. Certain embodiments of the present invention provide compositions for water-soluble formulations of estradiol metabolites, estradiol metabolite analogs, or estradiol metabolite prodrugs with increased half-lives that may be delivered parenterally or orally.

The second strategy to achieve sustained exposure to estradiol metabolite therapeutics is to incorporate estradiol metabolites, estradiol metabolite analogs, or estradiol metabolite prodrugs into some polymeric sustained release delivery system. Sustained release devices may incorporate estradiol metabolites, estradiol metabolite analogs, or estradiol metabolite prodrugs, themselves with increased plasma half-lives over that of the parent compounds. Of particular advantage in the manufacturing of sustained release delivery systems incorporating estradiol metabolites is the ability to modify the physical and chemical properties of the metabolite, such as by esterification, in order to optimize the release properties of the final delivery system. In certain embodiments of the present invention, formulations may consist of a mixture of estradiol metabolites, estradiol metabolite analogs, or estradiol metabolite prodrugs and another compound that forms a liquid at body temperature, such as in an eutectic mixture. In other embodiments, formulations are described that consist of a reservoir and permeation aids for the transdermal delivery of estradiol metabolites, estradiol metabolite analogs or estradiol metabolite prodrugs thereof. In yet other embodiments, the invention describes the composition of polymer matrices for the controlled release of estradiol metabolites, estradiol metabolite analogs, or estradiol metabolite prodrugs thereof. Such matrices may be composed of polymer microparticles or nanoparticles. In particular embodiments, such polymers are biodegradable.

### Estradiol metabolites

In certain embodiments, the estradiol metabolite is a catecholestradiol such as 2-hydroxyestradiol (Estra-1,3,5 (10)-triene-2,3,17-triol (17β)) or 4-hydroxyestradiol (Estra-1,3,5 (10)-triene-3,4,17-tnol (17β)) or a methoxyestradiol, such as 2-methoxyestradiol (Estra-1,3,5 (10)-triene<2-methoxy-3,17-diol (17β)) or 4-methoxyestradiol (Estra-1,3,5 (10)-triene-4-methoxy-3,17-diol (17β). Commercial preparations of all of these compounds are readily available. In addition, a method of producing a highly purified 2-methoxyestradiol is disclosed in U.S. Provisional Patent Application No. 150,293.

In certain embodiments, the estradiol metabolite may be attached to a hydrophilic polymer. The hydrophilic polymer may be selected from the group consisting of poly(propylene glycol), poly(ethylene glycol), copolymers of poly(ethylene glycol) and poly(propylene glycol). In particular embodiments the hydrophilic molecule is poly(ethylene glycol) (PEG).

In an alternative embodiment, the estradiol metabolite is associated with microparticles or nanoparticles. In certain embodiments, the microparticles or nanoparticles selected from the group consisting of poly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, polyurethanes, blends and copolymers thereof. In a particular embodiment, the microparticle or nanoparticle is poly(lactide - co-glycolide) (PLGA). In another particular embodiment, the microparticle or nanoparticle is composed of a biodegradable polymer.

The skilled artisan will realize that the compounds listed above are exemplary only and that many variations may be used, depending on the particular hydroxylation or methylation site on the parent estradiol compound. For example, estradiol can be hydroxylated or methylated at many sites and such variations are known in the art.

### Esters of Estradiol Metabolites

In certain embodiments, esters of estradiol metabolites are utilized to create prodrugs. The ester linkage stays intact during preparation and storage of the drug, only becoming vulnerable to hydrolysis after administration to a patient. Therefore, esters are optimal prodrugs because a physiological environment has abundant endogenous esterases to catalyze hydrolysis of the linkage. Once hydrolysis occurs, only the active estradiol metabolite and a non-toxic biological compound remain, such as acetic acid or propionic acid, for example.

In a certain embodiment, esters of estradiol metabolites are utilized to control solubility of the estradiol metabolites. Water solubility may be conferred by esterifying with succinic acid, for example. Other esters may improve solubility in a variety of other solvents, and may also allow some interaction between an estradiol metabolite ester and some polymer comprising a sustained release delivery device, which, in turn, would control the release of the ester prodrug from the polymer matrix and into the surrounding tissue fluids.

In a particular embodiment, esters of 2-methoxyestradiol include, but are not limited to 3-benzoyl-2-methoxyestradiol, 17-benzoyl-2-methoxyestradiol, 17-acetyl-2-methoxyestradiol, 3-acetyl-2-methoxyestradiol, 3,17-benzoyl-2-methoxyestradiol and 3,17-diacetyl-2-methoxyestradiol.

In another particular embodiment esters of 4-methoxyestradiol include, but are not limited to 3-benzoyl-4-methoxyestradiol, 17-benzoyl-4-methoxyestradiol, 17-acetyl-4-methoxyestradiol, 3-acetyl-4-methoxyestradiol, 3,17-dibenzoyl-4-methoxyestradiol and 3,17-diacetyl-4-methoxyestradiol.

In an alternate particular embodiment, esters of 2-hydroxyestradiol include, but are not limited to, 3-benzoyl-2-hydroxyestradiol, 17-benzoyl-2-hydroxyestradiol, 17-acetyl-2-hydroxyestradiol, 3-acetyl-2-hydroxyestradiol, 3,17-dibenzoyl-2-hydroxyestradiol, 3,17-diacetyl-2-hydroxyestradiol, 2,3-dibenzoyl-2-hydroxyestradiol, 2,17-dibenzoyl-2-hydroxyestradiol, 2,17-diacetyl-2-hydroxyestradiol, 2,3-diacetyl-2-hydroxyestradiol, 2,3,17-tribenzoyl-2-hydroxyestradiol and 2,3,17-triacetyl-2-hydroxyestradiol.

In another particular embodiment, esters of 4-hydroxyestradiol include, but are not limited to, 3-benzoyl-4-hydroxyestradiol, 17-benzoyl-4-hydroxyestradiol, 17-acetyl-4-hydraxyestradiol, 3-acetyl-4-hydroxyestradiol, 3,17-dibenzoyl-4-hydroxyestradiol, 3,17-diacetyl-4-hydroxyestradiol, 3,4-dibenzoyl-4-hydroxyestradiol, 4,17-dibenzoyl-4-hydroxyestradiol, 4,17-diacetyl-4-hydroxyestradiol, 3,4-diacetyl-4-hydroxyestradiol, 3,4,17-tribenzoyl-4-hydroxyestradiol and 3,4,17-triacetyl-4-hydroxyestradiol.

In a certain embodiment, esters of all four estradiol metabolites may be organic acid derivatives of the original estradiol metabolite. Particular embodiments include, but are not limited to, esters of propionic acid, butyric acid, valeric acid, hexanoic acid, benzoic acid, acetic acid, propionic acid, butyric acid, stearic acid and other fatty acids.

Estradiol metabolites of use in the present invention may be selected from the group consisting of 2-methoxyestradiol, 2-hydroxyestradiol, 4-methoxyestradiol and 4-hydroxyestradiol. In a particular embodiment, the ester derivative of an estradiol metabolite is selected from the group consisting of 3-benzoyl-2-methoxyestradiol; 17-benzoyl-2-methoxyestradiol; 17-acetyl-2-methoxyestradiol; 3-acetyl-2-methoxyestradiol; 3,17-dibenzoyl-2-methoxyestradiol; 3,17-diacetyl-2-methoxyestradiol; 3-benzoyl-4-methoxyestradiol; 17-benzoyl-4-methoxyestradiol; 17-acetyl-4-methoxyestradiol; 3-acetyl-4-methoxyestradiol; 3,17-dibenzoyl-4-methoxyestradiol; 3,17-diacetyl-4-methoxyestradiol; 3-benzoyl-2-hydroxyestradiol; 17-benzoyl-2-hydroxyestradiol; 17-acetyl-2-hydroxyestradiol; 3-acetyl-2-hydroxyestradiol; 3,17-dibenzoyl-2-hydroxyestradiol; 3,17-diacetyl-2-hydroxyestradiol; 2,3-dibenzoyl-2-hydroxyestradiol; 2,17-dibenzoyl-2-hydroxyestradiol; 2,17-diacetyl-2-hydroxyestradiol; 2,3-diacetyl-2-hydroxyestradiol; 2,3,17-tribenzoyl-2-hydroxyestradiol; 2,3,17-triacetyl-2-hydroxyestradiol; 3-benzoyl -4-hydroxyestradiol; 17-benzoyl-4-hydroxyestradiol; 17-acetyl-4-hydroxyestradiol; 3-acetyl-4-hydroxyestradiol; 3,17-dibenzoyl-4-hydroxyestradiol; 3,17-diacetyl-4-hydroxyestradiol; 3,4-dibenzoyl-4-hydroxyestradiol; 4,17-dibenzoyl-4-hydroxyestradiol; 4,17-diacetyl-4-hydroxyestradiol; 3,4-diacetyl-4-hydroxyestradiol; 3,4,17-tribenzoyl-4-hydroxyestradiol; 3,4,17-triacetyl-4-hydroxyestradiol.

Methods for synthesizing several esters of estradiol metabolites are known. (See, *e.g.*, Japanese Patent NO. 57,041,479 and 49,100,070).

In an alternative embodiment, the ester of an estradiol metabolite may be attached to a hydrophilic polymer. A hydrophilic polymer increases the half-life of the compound and also allows for less frequent and lower dose administrations. In certain embodiments, a hydrolysable linkage is included to free the ester molecule from the hydrophilic polymer after hydrolysis. This will allow the ester molecule to enter the cytoplasm of cells only after hydrolysis as it is slower or unable to pass through a cell membrane with a hydrophilic molecule, such as PEG, attached.

In certain embodiments, the ester of an estradiol metabolite, with or without a hydrophilic polymer attached, may also be incorporated in microparticles, such as microspheres or nanospheres.

The skilled artisan will realize that the compounds listed above are exemplary only and that many variations may be used, depending on the particular ester derivative created from a particular estradiol metabolite. Such variations are known in the art.

### Preparation of Intravenous or Oral Formulations of Estradiol Metabolites

In certain embodiments, estradiol metabolites, estradiol metabolite analogs, or estradiol metabolite prodrugs are derivatized with polar or ionic compounds, such that the derivative is water soluble and exhibits prolonged plasma circulation times compared to the parent metabolite. In a particular embodiment, estradiol metabolites, estradiol metabolite analogs, or estradiol metabolite prodrugs are derivatized with dicarboxylic acid compounds, including but not limited to oxalic, malonic, maleic, succinic, glutaric, adipic, pimelic, pamoic or other diacids. In another particular embodiment, diacids with shorter intervening carbon chains, such as succinic, glutaric, maleic, malonic, or oxalic acids are used. Compounds such as these confer increased water solubility and increased plasma circulation times when combined with estradiol metabolites. Methods of esterification with diacids may be effected with the appropriate anhydride or mixed anhydride of the diacid using techniques well known in the art. The invention includes all modifications to estradiol metabolites, estradiol metabolite analogs, or estradiol metabolite prodrugs that achieve increased water solubility and plasma lifetime. Such derivatives may be conceived and synthetic pathways for such derivatives may be readily executed by those with ordinary skill in the art (e.g. US Patent NO 2,897,218). Any available functional group on the estradiol metabolite or estradiol metabolite ester may be derivatized.

### Preparation of Transdermal Formulations of Estradiol Metabolites

In certain embodiments, estradiol metabolites, estradiol metabolite analogs, or estradiol metabolite prodrugs thereof are mixed with appropriate vehicles comprising a reservoir from which the drug can partition into the skin at an appropriate rate. The drug must diffuse through the protective stratum corneum barrier before entering the dermal layer, from which systemic drug absorption takes place. Permeation may be enhanced by physical means, such as increased hydration, application of ultrasound, thermal, or electrical potentials, or by chemical means, such as incorporation of fatty acid esters, chaotropic agents, polyols, terpenoids, or surfactants. Partitioning between the vehicle and stratum corneum depends on the relative solubility of the drug in each environment. Thus, both the identity of the vehicle and composition of the drug, via esterification, for example, may be changed to optimize the release profile of the drug from the transdermal patch. In a particular embodiment, solutions or solid suspensions of estradiol metabolite are made in media containing skin penetration enhancers and/or biocompatible solvent or mixtures of solvents or a transdermal adhesive. The suspension is designed to promote the dissolution into and transdermal permeation of the drug through the stratum corneum. In another particular embodiment, estradiol metabolites, estradiol metabolite analogs, or estradiol metabolite prodrugs are dissolved in a vehicle such as menthol, so that the formulation is liquid at skin temperature. In another particular embodiment estradiol metabolites, estradiol metabolite analogs, or estradiol metabolite prodrugs are suspended or dissolved in an appropriate transdermal adhesive and incorporated into a standard drug in adhesive transdermal patch; or incorporated into a multilayered patch comprising drug in adhesive (next to the skin), a rate controlling membrane, and drug in adhesive serving as a reservoir.

### Preparation of Sustained Release Microparticle Formulations of Estradiol Metabolites

Hepatic first pass metabolism decreases the bioavailability of orally delivered steroids. As a result, steroids are often given by injection. Steroids are typically short acting, and chronic treatment requires repeated injections. Formation of estradiol metabolite or estradiol metabolite analog prodrugs by esterification of steroid and derivatation or parenteral delivery in an oil vehicle decrease dosing frequency. An alternative way to achieve therapeutic levels of estradiol metabolites for a prolonged period of time is to incorporate estradiol metabolites, estradiol metabolite analogs or estradiol metabolite prodrugs into extended delivery devices.

A certain embodiment of the present invention provides microspheres composed of poly-D,L-(lactide-co-glycolide). This polyester is biocompatible, with a long record of medical safety. Polymer erosion in the body controls the rate of drug release and one skilled in the art is adept at manipulating this rate. Extended release dosing systems are an ideal compliment to estradiol metabolite treatments for conditions such as type II diabetes, which often require lifelong drug therapy.

The invention further provides methods for encapsulating estradiol metabolites, estradiol metabolite analogs, or estradiol metabolite prodrugs in suitable polymeric delivery devices for sustained release. Drug may be dispersed through the polymer matrix, or alternatively, the drug, either alone or as a mixture with another polymer, solvent, or other agent may be surrounded by a polymeric capsule. Release of the drug may be controlled by diffusion through the polymer matrix, or by a combination of drug diffusion through the polymer matrix and erosion of the polymeric delivery device. Preferred devices include rods, stints, pellets, discs, lozenges, wafers, capsules, films, microparticles, or nanoparticles, microcapsules, or nanocapsules. In a particular embodiment, estradiol metabolites, estradiol metabolite analogs, or estradiol metabolite prodrugs are associated with a biodegradable polymer in microparticle or nanoparticle form.

In certain embodiments, the estradiol metabolites, estradiol metabolite analogs, or estradiol metabolite prodrugs are associated with a polymer in a microparticle form. In a preferred embodiment, a microparticle has a preferred diameter of less than 1.0 mm and is preferably between 1.0 and 200.0 micrometers. Microparticles include both microspheres and microcapsules. Microspheres are typically solid spherical microparticles and microcapsules are microspheres with a core of a different polymer, drug or composition.

In certain embodiments, the estradiol metabolites, estradiol metabolite analogs, or estradiol metabolite prodrugs, with or without a hydrophilic polymer attached, are associated with biodegradable submicron particles for controlled release of the metabolite molecules. A nanoparticle has a diameter ranging from 20.0 nanometers to about 2.0 microns and is typically between 100.0 nanometers and 1.0 micron.

Nanoparticles can be created by any technique well known in the art. They can be created in the same manner as microparticles, except that high-speed mixing or homogenization is used to reduce the size of the polymer/bioactive agent emulsions to less than 2.0 microns and preferably below 1.0 micron. (See, e.g., WO 97/04747)

In certain embodiments, the microparticles or nanoparticles are comprised of poly(lactide)s, poly(glycolide)s, poly(lactide-co-glycolide)s, poly(lactic acid)s, poly(glycolic acid)s, poly(lactic acid-co-glycolic acid)s, polyeaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, biodegradable polyurethanes, blends and copolymers thereof.

In another embodiment, the microparticle or nanoparticle is poly(lactide -co-glycolide) (PLGA). PLGA degrades when exposed to physiological pH and hydrolyzes to form lactic acid and glycolic acid, which are normal byproducts of cellular metabolism. The disintegration rate of PLGA polymers will vary depending on the polymer molecular weight, ratio of lactide to glycolide monomers in the polymer chain, and stereoregularity of the monomer subunits. Polymer disintegration rates will be increased by mixtures of L and D stereoisomers that disrupt the polymer crystallinity. In addition, microspheres may contain blends of two or more biodegradable polymers, of different molecular weight and/or monomer ratio.

In other alternative embodiments, derivatized biodegradable microparticles, including hydrophilic polymers attached to PLGA, can be used to form microspheres.

The illustrative embodiments describe methods for the encapsulation of estradiol metabolites, estradiol metabolite analogs, or estradiol metabolite prodrugs. Methods may be chosen and adapted based on several considerations including solubility of the estradiol metabolite in a particular solvent, desired physical state of the drug in the final delivery system, desired drug loading in the microsphere delivery system, desired release rate and duration of release of the drug from the delivery system, desired particle size, and so forth. Microspheres can be made by any technique well known in the art. In certain embodiments, microspheres are produced by single or double emulsion steps followed by solvent removal. In alternative embodiments, other known methods such as spray drying, solvent evaporation, phase separation and coacervation may be utilized to create microspheres. Other methods and variations of the above are also known in the art and may also be used with the present invention.

In a particular embodiment, polymeric microparticles are formed by spray drying a solution of polymer and estradiol metabolites, estradiol metabolite analogs, or estradiol metabolite prodrugs dissolved in an appropriate organic solvent. The concentrations of the polymer and solvent are controlled to give microparticles that contain a predetermined weight ratio of drug to polymer. The core load, in part, controls the release properties of that particular drug from the delivery device.

In another particular embodiment, polymeric microparticles are formed by wet emulsification followed by solvent removal. Drug and polymer are dissolved in a suitable organic solvent that will comprise the discontinuous, dispersed phase of the emulsion. In certain embodiments, the discontinuous phase solvent will also contain a preservative, such as an antioxidant, buffer, or other agent intended to preserve the chemical integrity of the microparticle components. The preservative may be dissolved or suspended in the discontinuous phase solvent. The organic solvent chosen should be capable of solubilizing sufficient drug and polymer to obtain a solution that can form microspheres when mixed with a continuous phase, and subsequently one that can form microparticles upon removal of the solvent after dispersion of the discontinuous phase in the continuous phase. For purposes of solubilizing sufficient quantities of both drug and polymer, a combination of solvents may be used. In particular embodiments, a quantity of a second solvent containing the drug is mixed with a solvent containing the polymer. The second solvent is sufficiently miscible with the first solvent, so that a clear, homogeneous solution is formed upon mixing the two discontinuous phase solvents. The second solvent may be immiscible, partly miscible, or completely miscible with the continuous phase solvent.

The discontinuous phase solvent or solvent mixture may be immiscible, or partly miscible with the continuous phase solvent. In particular embodiments employing a single discontinuous phase solvent, that solvent may be between 0.05% and 20% miscible with the continuous phase solvent. In another particular embodiment, the discontinuous phase solvent will be between 1% and 10% miscible in the continuous phase solvent.

The discontinuous phase solvent is mixed with a continuous phase liquid containing appropriate emulsion stabilizers, as necessary to form an emulsion. The continuous phase liquid is typically not a solvent for either the polymer or the encapsulated drug. The continuous phase may, however, be a solvent for the discontinuous phase solvent or solvents. The volume or mass ratio of discontinuous phase solvent to continuous phase solvent during emulsification may be any ratio that allows microparticles to be formed with the desired characteristics including particle size, and physical state of the drug encapsulated within the microparticles, for example. In a particular embodiment, the discontinuous phase to continuous phase volume ratios may range from 0.5:1 to 30:1. In certain embodiments, the continuous phase may contain from trace to saturating amounts of the discontinuous phase solvent or a mixture of solvents designed to modulate the extraction of discontinuous solvent or solvents from the oil phase of the emulsion. In a particular embodiment, the continuous phase liquid is water.

Emulsifiers may be added to the continuous phase liquid to stabilize the emulsion during formation and subsequent discontinuous phase solvent removal. Examples of such emulsifiers include, phospholipids, such as lecithin, ionic and nonionic surfactants, poloxamers, or polymers such as polyvinyl pyrrolidone and polyvinyl alcohol. In preferred embodiments, polyvinyl alcohol is used in concentrations ranging from 0.05 to 10% w/v. In a particularly embodiment, polyvinyl alcohol is used in concentrations between 0.3 and 4% w/v in the aqueous phase.

In wet emulsification, particle size is controlled in part by the type and amount of emulsifier contained in the aqueous phase, and also by the mixing energy used to disperse the discontinuous phase into the continuous phase. Mixing may be effected by any of various means including rapid stirring of the phases in a single vessel using a magnetic bar, impeller device, and rotor-stator homogenizer, or probe or bath sonicators. In a particular embodiment, mixing is achieved by stirring with a magnetic bar.

Solvent removal from the discontinuous phase of the emulsion and consequent hardening of microparticles may be achieved by various means. The emulsion may be held without extracting, at a predetermined temperature for a defined period of time prior to solvent extraction. Or alternatively, the organic solvent may be extracted immediately upon emulsification.

In certain embodiments of the present invention, solvent removal may be controlled by evaporation. In other embodiments, the evaporation may be assisted by application of reduced pressure. In a certain embodiment, the solvent may be partly miscible with water, such that after emulsification, rapid hardening of microparticles will result from the addition of a sufficient further quantity of water to the emulsion to solubilize all of the organic solvent contained in the emulsion. The rate at which the extraction medium is added to the emulsion, or the rate at which the emulsion is added to the extraction medium may vary depending on the desired solvent extraction rate, which is in turn dependent on the sensitivity of the drug/polymer system to changes in solution conditions. One of skill in the art is capable of determining these factors. In a further embodiment, discontinuous phase solvent extraction may be achieved by modulating the temperature of the emulsion so that the solubility of the discontinuous phase in the continuous phase increases to sufficiently extract the discontinuous phase solvent.

### Examples

It should be appreciated by those skilled in the art that the techniques disclosed in the examples which follow represent techniques discovered by the inventors to function well in the practice of the invention, and thus can be considered to constitute the preferred modes for its practice. However, those of skill in the art should appreciate, in light of the present disclosure, that many changes can be made in the specific embodiments disclosed herein which will still obtain a like or similar result without departing from the spirit and scope of the invention.

### Example 1. Encapsulation of 2-methoxyestradiol into poly-(lactide-co-glycolide) microspheres.

1.6 g of poly-(lactide-co-glycolide) (PLGA) with a 1:1 mole ratio of lactide to glycolide monomer and with an intrinsic viscosity of 0.15 dl/g (PLGA 5050 2A, Medisorb, USA), and 800 mg 2-methoxyestradiol (2ME) were dissolved in 28 ml ethyl acetate by heating at 65°C. This oil phase was slowly poured into 80 ml of aqueous polyvinyl alcohol (av. Mol. Wt. 100 kD, 1% w/v) in a 250 ml beaker containing a magnetic bar stirring at 450 rpm. The mixture was thus emulsified for 5 min. before the emulsion was rapidly poured into 600 ml of 1% w/v aqueous polyvinyl alcohol. The microspheres were allowed to harden for 3 hr. by magnetic stirring at room temperature and ambient pressure. The hardened particles were collected and washed with water by centrifugation and then lyophilized.

A sample of dry microspheres was dissolved in dimethyl sulfoxide (DMSO) and the 2ME present in the sample was quantified by HPLC analysis against standard concentrations of the drug. The core load was 28.0% 2ME by weight. Encapsulation efficiency was calculated as the percent ratio of measured core load to nominal core load, and was 85% for this preparation.

18 mg 2ME microspheres, containing 5 mg 2ME, were suspended in a vehicle consisting of 0.25 ml sodium carboxymethylcellulose, 2.5% by weight. The suspension was injected subcutaneously into Sprague Dawley rats. Three animals were sacrificed at specified time points, injection sites were dissected, and blood samples were withdrawn, and plasma was separated and frozen at -80°C. Microsphere implants were isolated from the injection sites and extracted with DMSO to quantify the unreleased drug by HPLC. The in vivo release was calculated by subtracting the amount of drug remaining in the implants from the total amount of 2ME injected. The in vivo release profile shows a burst release of approximately 35% in the first day, followed by linear release of 100% of the encapsulated drug in 28 days.

The frozen plasma samples were thawed, extracted, derivatized, and plasma levels of 2ME were quantified by gas chromatography against known 2ME standards. One day after injection, plasma 2ME levels were 6.5 ng/ml, dropping to 5 ng/ml at day three. 2ME plasma concentrations then increased between day three and day seven, and were sustained at 8 ng/ml through day 14. Plasma concentrations of the drug then steadily decreased between day 14 and day 28 to a final concentration of 2 ng/ml at day 28.

### Example 2. Encapsulation of 2-hydroxyestradiol into poly.(lactide-co-glycolide) microspheres.

A microsphere preparation was made by dissolving 1067 mg 2-hydroxyestradiol (2HE) and 1600 mg PLGA (50:50 lactide:glycolide, Mw 27 kD) in 28 ml ethyl acetate. The microspheres were prepared according to the details in Example 1. The core load was measured to be 38.3%, 96% encapsulation efficiency.

Microspheres equivalent to 5 mg 2HE were injected subcutaneously into rats. At predetermined intervals, animals were sacrificed and injection sites were dissected to recover microspheres. At the same time, blood samples were taken from the animals, and the plasma was separated and frozen at -80°C. The recovered microspheres were cleaned by centrifugation and lyophilized. Carefully weighed samples were dissolved in DMSO and the 2HE content of the recovered microspheres was quantified by HPLC analysis. Release of estradiol metabolites in vivo was calculated indirectly by subtracting the amount of drug remaining in microspheres after in vivo incubation from the initial amount of drug in the microspheres. 32% of the encapsulated drug was released within one day of injection, with approximately 50% of the dose released after three days. The remaining drug was released steadily between day 3 and day 28 post-injection.

After the samples from each time point were collected, the frozen plasma samples were thawed, extracted, derivatized, and plasma levels of 2HE and 2ME were quantified by gas chromatography against known standards. The plasma pharmacokinetic profile showed a blood level of approximately 20 ng/ml 2HE, 24 hours post-injection, which decreased steadily to day 7 post-injection. Blood levels of 2HE were detectable between trace levels and 2 ng/ml between day 7 and day 28. A lower burst level of 2ME was detected (11.5 ng/ml) one day after injection, which trailed off through day 7 to 2 ng/ml, which was sustained between day 7 and day 28.

### Example 3. Encapsulation of 2HE in PLGA microspheres by a selective solvent extraction method.

Poly-D,L- (lactic-co-glycolic) acid in a 50:50 mole ratio (PLGA 5050 2.5M) with an average molecular weight of 27 kD was dissolved in ethyl acetate to a concentration of 20% w/v. A second solution was made by dissolving 300 mg 2-hydroxyestradiol in 1.2 ml dimethyl sulfoxide (DMSO). The two solutions were mixed by vortexing, resulting in a single, clear solution. This organic solution was emulsified with an aqueous phase consisting of 17.5 ml water containing 700 mg polyvinyl alcohol, 2.5 ml ethyl acetate, and 4 ml DMSO in a 50 ml beaker, by stirring with a 1 inch magnetic bar at 650 rpm for 5 min at 4°C. The resulting emulsion was slowly poured into a 600 ml beaker containing 240 ml water, 48 ml DMSO, and 6 ml ethyl acetate at 4°C. The particle suspension was allowed to warm to room temperature under ambient conditions and ethyl acetate was allowed to extract/evaporate from the emulsion overnight. The hardened particles were filtered, washed with water, and air dried prior to determining core load.

Dried microparticles from each preparation were solubilized in DMSO and quantified by HPLC against 2HE standards. The 2HE content was found to be 13.1 %. Encapsulation efficiency was 66%.

Microspheres equivalent to 5 mg 2HE were injected subcutaneously into rats. At predetermined intervals, animals were sacrificed and injection sites were dissected to recover microspheres. At the same time, blood samples were taken from the animals, and the plasma was separated and frozen at -80°C. The recovered microspheres were cleaned by centrifugation and lyophilized. Carefully weighed samples were dissolved in DMSO and the 2HE content of the recovered microspheres was quantified by HPLC analysis. Release of estradiol metabolites in vivo was calculated indirectly by subtracting the amount of drug remaining in microspheres after in vivo incubation from the initial amount of drug in the microspheres. In vivo release of2HE was characterized by a burst release of 38% of the dose during the first day post-injection, followed by cumulative release of 55% at day 3 post-injection. 30% of the total dose was released between day 3 and day 28, with a total of 88% of the dose released in 4 weeks.

After the samples from each time point were collected, the frozen plasma samples were thawed, extracted, derivatized, and plasma levels of 2HE and 2ME were quantified by gas chromatography against known standards. The 38% burst release corresponded to a plasma level of 4 ng/ml 2HE, which decreased to trace levels between day 3 and day 28. Plasma levels of 2ME peaked at 5.5 ng/ml one day post-injection, and decreased to 2 ng/ml at day 3. This level was sustained between day 3 and day 28.

### Example 4. Solid-in-oil-in water encapsulation of 2ME

DMSO solutions of 2ME at a 25% w/v concentration were quench frozen in liquid nitrogen to prevent the crystallization of the drug. One ml aliquots of the frozen 2ME-DMSO solution were crushed in a cold mortar and pestle then mixed at 33,000 rpm with 10 ml of chilled 20% w/v PLGA 50:50 (lactide:glycolide, average Mw 53kD) solution in ethyl acetate, using a Fisher Powergen 125 rotor/stator homogenizer fitted with a chilled 7 mm tip. Two preparations were made, the first was emulsified with all components chilled to -20°C, and the second chilled to 4°C. Both temperatures maintained the 2ME-DMSO solution in the frozen state. The solid-in-oil emulsions were added to 30 ml distilled water containing 4% w/v polyvinyl alcohol and 3.5 ml ethyl acetate at 4°C stirring at 600 rpm in a 100 ml beaker with a 1 inch stir bar. The resulting solid-in-oil-in water emulsion was slowly poured into 250 ml ice cold water in a 600 ml beaker containing 10 ml ethyl acetate, and stirred at 400 rpm with a 1,5 inch stir bar. The extraction beaker was placed into an ice bath during microsphere hardening. The beaker was stirred overnight to extract the solvents from the microspheres, and the temperature was allowed to increase to 22°C slowly as the ice melted and water warmed under ambient conditions.

The particles were collected by filtration, washed with water and lyophilized. Dried particles were dissolved in DMSO and the 2ME content was measured by HPLC. Encapsulation efficiency of the solid/oil/water technique at -20°C and 4°C is presented in Table I.

**Table I. 2ME encapsulation efficiency using the solid-in-oil-in-water technique.**

| Emulsification Temperature, °C | Nominal Core Load, % | Measured Core Load, % | Encapsulation Efficiency, % |
|---|---|---|---|
| -20 | 11.1 | 6.7 | 60 |
| 4 | 11.1 | 9.3 | 84 |

In vitro release of 2ME was measured by adding 12.5 mg microspheres to 100 ml 50% aqueous ethyl alcohol. Aliquots were removed at predetermined intervals and 2ME concentrations were measured by UV absorbance spectroscopy. The preparation that was emulsified at -20°C released 15 % of the encapsulated drug in a linear fashion for 6 hours. By contrast, the formulation that was emulsified at 4°C released 7% of the encapsulated drug within 30 minutes, with the release rate steadily decreasing between 30 minutes and 7 hours.

### Example S. Eutectic mixture of 2ME and menthol.

For chronic administration of low dose drugs, a transdermal patch may be preferable to repeated injections of PLGA microspheres. Two key obstacles to transdermal administration are formulation of a stable, high concentration reservoir of drug, and some mechanism to enhance skin permeability. Kaplun-Frischoff and Touitou (1997) J. Pharm. Sci. 86:1394-1399 (Testosterone skin permeation enhancement by menthol through formation of eutectic with drug and interaction with skin lipids) found that testosterone formed a eutectic mixture with menthol, which is a known skin permeability enhancer. However, because 2ME has a much lower solubility in alcohols than does testosterone, formation of a eutectic mixture of 2ME and menthol is not an obvious extension of the current art.

187.6 mg of menthol was ground in a mortar and pestle with 121.3 mg 2ME for five minutes, to form a homogeneous, waxy solid. 1.2 mg of this mixture was hermetically sealed in an aluminum pan and loaded into the sample compartment of a TA Instruments Q10 differential scanning calorimeter at 25°C. The sample was scanned from 25 to 160°C at 10°C/min. A first-order endothermic peak was noted with an onset temperature of 31.47°C. This peak does not correspond to either of the pure compounds (menthol 41.77°C, 2ME 187.36°C), and is consistent with the formation of a separate, eutectic phase. Transdermal delivery of the estradiol metabolite is expected to be enhanced from the eutectic mixture with menthol, since the mixture will be liquid at body temperature.

### Example 6. 3-benzoyl-2-methoxyestradiol microspheres prepared by spray drying

536 mg 3-benzoyl-2methoxyestradiol and 1250 mg PLGA (50:50 lactide:glycolide, 27kD Mw) were dissolved in 25 ml methylene chloride. The solution was pumped at 3 ml/mm, through the inlet atomizer of a Buchi Mini Spray Dryer model B-191. Equipment settings were as follows: inlet temperature, 50°C; outlet temperature, 43°C; aspirator, 80%; atomizer air flow, 600 ml/mm. Particles were collected and examined by scanning electron microscopy (06/18/02). Particle size ranged between 0.2 and 3 um. No drug crystals were noted, indicating efficient encapsulation of the drug.

### Example 7. 2-methoxyestradiol microspheres containing an antioxidant, prepared by extrusion through a packed-bed emulsifier

800 mg PLGA (50:50 lactide:glycolide, 13 kD average Mw), 400 mg 2-methoxyestradiol, and 8.2 mg BHT were dissolved in 14 ml ethyl acetate at 65°C. This oil phase was emulsified with an aqueous phase consisting of 1% w/v polyvinyl alcohol at a ratio of 1 part oil to 1.5 parts water. The resulting emulsion was either pumped directly at 3 ml/min. into a solvent extraction medium consisting of 800 ml 0.5% w/v polyvinyl alcohol, or held at 65°C for approximately 10 min prior to adding the emulsion to the extraction medium, The microspheres were allowed to harden in the extraction medium by stirring at room temperature for 2 hours. The hardened microspheres were collected by centrifugation, washed with distilled water, and lyophilized.

Dried microparticles from this preparation were solubilized in DMSO and quantified by HPLC against 2ME standards. The 2ME content was found to be 33.2%. Encapsulation efficiency was 97%. BHT content of the microspheres was quantified in the same assay against known BHT standards. The BHT loading was 0.7% by weight, corresponding to 100% encapsulation efficiency for the preservative.

### Example 8. 2-methoxyestradiol microspheres prepared by temperature modulated solvent extraction.

1600 mg PLGA (50:50 lactide:glycolide, 13 kD Mw) and 805 mg 2-methoxyestradiol were dissolved in 28 ml ethyl acetate at 65°C. This oil phase was emulsified with an aqueous phase consisting of 1% w/v polyvinyl alcohol at a ratio of 1 part oil to 20 parts water. The resulting emulsion was pumped through a lag tube immersed in ice water. The solubility of ethyl acetate in water increases with decreasing temperature, such that the cooled aqueous phase becomes a reservoir with increasing capacity for the organic solvent. The cooled microsphere suspension was pumped into a beaker and the microspheres were hardened by stirring at room temperature for 3 hours. The hardened microspheres were collected by centrifugation, washed with distilled water, and lyophilized.

Dried microparticles from this preparation were solubilized in DMSO and quantified by HPLC against 2ME standards. The 2ME content was found to be 27.3%. Encapsulation efficiency was 82%.

### Example 9. Stabilization of 2-methoxyestradiol in microspheres by annealing.

Microspheres containing 2ME were prepared according to details in Example 6, above. The finished microsphere powder was mixed with a 10-fold excess by weight of granular sucrose. The solid suspension was sealed in a polypropylene tube and immersed in a 65°C water bath for 3 hours to allow crystallization of the encapsulated 2-methoxyestradiol. After annealing, the tube was removed from the temperature bath and sufficient water was added to dissolve the sucrose. The microspheres were washed three times with water by centrifugation, and lyophilized. Differential scanning calorimetry on a sample of the annealed microspheres confirmed that > 99% of the drug had crystallized.

Samples of microspheres before and after annealing were loaded into glass vials, sealed in ambient atmosphere, and placed into temperature controlled incubators at 23°C or 38°C. Vials were removed from incubators at predetermined time intervals and the content and purity of the 2ME in the microspheres was analyzed. The formulation containing amorphous drug degraded by more than 13% when stored at room temperature, whereas the Formulation containing crystalline drug did not degrade during an incubation of 58 days at 38°C.

**Table II. Storage stability of crystalline and amorphous 2ME in microspheres.**

| Lot # | 2MEcore load %w/w | % crystalline 2ME, time zero | Storage | purity |
|---|---|---|---|---|
| 050-049-SE | 26.9 | 17 | 27 days, 23°C | 87.4% |
| 041-158-B | 30.4 | 99.7 | 58 days, 38°C | 99.3% |

### Example 10. 2-methoxyestradiol microspheres prepared by encapsulation of micronized crystals.

2-methoxyestradiol drug substance was ground in a mortar and pestle until the particles were less than 5 um diameter. Particle size was monitored by scanning electron microscopy. The micronized drug, 129 mg, was added to 1 ml of a 30% w/v solution of PLGA (50:50 lactide:glycolide, 27kD Mw) in ethyl acetate. The drug was suspended in the polymer solution using a probe sonicator with the vessel placed on ice. The suspension was added dropwise to 50 ml of aqueous 4% w/v polyvinyl alcohol stirring at 800 rpm in a 150 ml beaker. The microsphere suspension was stirred overnight at room temperature. The hardened microspheres were collected by centrifugation, washed with distilled water, and lyophilized. Chromatographic analysis of a sample of the final microsphere preparation dissolved in dimethyl sulfoxide, and measured against known 2ME standards, showed that the core load was 28.8% 2-methoxyestradiol by weight, making the encapsulation efficiency 96%.

### Example 11. Preparation of 2-methoxyestradiol transdermal patch formulations.

2-methoxyest-adiol drug substance was suspended in different transdermal grade pressure sensitive adhesives (National Starch) and coated onto polyethylene and aluminum vapor coated polyester backings (3M) using a Gardco "Microm" film applicator. Coatings were dried overnight in a fume hood and drying completed in an 80 °C oven for 2-4 hours.

### Example 12. Preparation of 2-methoxyestradiol-3,17-diacetate microspheres.

400 mg of poly-(lactide-co-glycolide) (PLGA) with a 1:1 mole ratio of lactide to glycolide monomer and with an intrinsic viscosity of 0.25 dl/g, average Mw 27kD (PLGA 5050 2.5M, Medisorb, USA), and 200 mg 2-methoxyestradiol-3,17-diacetate were dissolved in 7 ml ethyl acetate by stirring at 23°C. This oil phase was slowly poured into 20 ml of aqueous polyvinyl alcohol (av. Mol. Wt. 100 kD, 1 % w/v) in a 50 ml beaker containing a magnetic bar stirring at 450 rpm. The mixture was thus emulsified for 5 min. before the emulsion was rapidly poured into 150 ml of 1% w/v aqueous polyvinyl alcohol. The microspheres were allowed to harden for 3 hr. by magnetic stirring at room temperature and ambient pressure. The hardened particles were collected and washed with water by centrifugation and then lyophilized.

A second preparation was made by dissolving 400 mg each of the PLGA and 2-methoxyestradiol-3,17-diacetate in 7 ml ethyl acetate. Emulsification was performed as described above.

A sample of dry microspheres from each preparation was dissolved in dimethyl sulfoxide (DMSO) and the 2ME diacetate present in the samples was quantified by HPLC analysis against standard concentrations of the drug. Drug loading and encapsulation efficiency are detailed in Table III.

**Table III. Drug loading for 2-methoxyestradiol-3,17-diacetate microspheres.**

| Lot # | Nominal Loading | Measured Loading | Encapsulation Efficiency |
|---|---|---|---|
| 041-034-A | 33.3% | 29.8% | 89% |
| 041-034-B | 50.0% | 44.1% | 88% |

Microspheres from each preparation equivalent to 5 mg 2ME diacetate were added to 100 ml of 50% aqueous alcohol. The vessels were stirred at 100 rpm at 23°C for 7 hours. Samples of the release medium were withdrawn at intervals, and the concentration of the drug was measured by UV absorbance at 287 nm. Lot 041-034-A released 5% of the encapsulated drug in 1 hr, with 8% total release in 7 hours in the in vitro release assay. In contrast, 8% of the encapsulated drug was released within 30 min. from lot 041-034-B, with a subsequent total of 24% released in 7 hours.

Microspheres equivalent to 5 mg 2ME diacetate from lot# 041-034-B described above, were injected subcutaneously into Sprague Dawley rats. Blood was collected periodically over 4 weeks. Immediately after collection, plasma was separated and stored frozen at -80°C. After the samples from each time point were collected, the frozen plasma samples were thawed, extracted, derivatized, and plasma levels oF2ME were quantified by gas chromatography against known 2ME standards. The pharmacokinetic profile was characterized by a steady increase to 2 ng/ml between day 0 and day3. Plasma levels were sustained between 1 and 3 ng/ml between day 3 and day 28. Interestingly, there was no large burst release of 2-methoxyestradiol observed during the first three days after injection from microsphere formulations containing the diacetate prodrug.

All of the COMPOSITIONS, METHODS and APPARATUS disclosed and claimed herein can be made and executed without undue experimentation in light of the present disclosure. While the compositions and methods of this invention have been described in terms of preferred embodiments, it will be apparent to those of skill in the art that variations may be applied to the COMPOSITIONS, METHODS and APPARATUS and in the steps or in the sequence of steps of the methods described herein without departing from the concept, spirit and scope of the invention. More specifically, it will be apparent that certain agents that are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

## Claims

1. A composition of matter comprising:
(a) an estradiol metabolite; and
(b) a material providing for sustained release.

2. The composition of claim 1, wherein said material providing for sustained release is selected from the group consisting of microparticles, nanoparticles, patches, crystals, gels, rods, stints, pellets, discs, lozenges, wafers, capsules, films, microcapsules, nanocapsules, hydrogels, liposomes, implants and vaginal rings.

3. The composition of claim 2, wherein said microparticles or nanoparticles are comprised of a biodegradable polymer selected from the group consisting poly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, polyurethanes, blends and copolymers thereof.

4. The composition of claim 3, wherein said microparticles or nanoparticles are comprised of poly(d,l-lactide-co-glycolide).

5. The composition of claim 2, wherein said microparticles have a diameter between 1 and 200 micrometers.

6. The composition of claim 2, wherein said nanoparticles have a diameter between 20 and 2000 nanometers.

7. The composition of claim 2, wherein said microparticles or nanoparticles further comprise an additive.

8. The composition of claim 7, wherein said additive is selected from the group consisting of butylated hydroxytoluene, propyl gallate, a tocopherol, ascorbyl palmitate, an antioxidant, a release modifier and a buffer.

9. The composition of claim 1, wherein said estradiol metabolite is selected from the group consisting of 2-methoxy estradiol, 2-hydroxy estradiol, 4-methoxy estradiol and 4-hydroxy estradiol.

10. The composition of claim 1, wherein said estradiol metabolite is delivered transdermally,

11. The composition of claim 10, wherein said transdermal delivery is selected from the group consisting of buccal, oral, ocular, nasal, rectal and vaginal.

12. The composition of claim 1, wherein said estradiol metabolite is a prodrug.

13. The composition of claim 12, wherein said prodrug is an ester.

14. The composition of claim 13, wherein said ester is selected from the group consisting of 3-benzoyl-2-methoxy estradiol; 17-benzoyl-2-methoxy estradiol; 17-acetyl-2-methoxy estradiol; 3-acetyl-2-methoxy estradiol; 3,17-dibenzoyl-2-methoxy estradiol; 3,17-diajcetyl'2-methoxy estradiol; 3-benzoyl-4-methoxy estradiol; 17-benzoyl-4-methoxy estradiol; 17-acetyl-4-methoxy estradiol; 3-acetyl-4-methoxy estradiol; 3,17-dibenzoyl-4-methoxy estradiol; 3,17-diacetyl-4-mothoxy estradiol; 3-benzoyl -2-hydroxy estradiol; 17-benzoyl-2-hydroxy estradiol; 17-acetyl-2-hydroxy estradiol; 3-acetyl-2-hydroxy estradiol; 3,17-dibenzoyl-2-hydroxy estradiol; 3,17-diacetyl-2-hydroxy estradiol; 2,3-dibenzoyl-2-hydroxy estradiol; 2,17-dibenzoyI-2-hydrvxy estradiol; 2,17-diacetyl-2-hydroxy estradiol; 2,3-diacetyl-2-hydroxy estradiol; 2,3,17-tribenzoyl-2-hydroxy estradiol; 2,3,17-triacetyl-2-hydroxy estradiol; 3-benzoyl-4-hydroxy estradiol; 17-benzoyl-4-hydroxy estradiol; 17-acetyl-4-hydroxy estradiol; 3-acetyl-4-hydroxy estradiol; 3,17-dibenzoyl-4-hydroxy estradiol; 3,17-diacetyl-4-hydroxy estradiol; 3,4-dibenzoyl-4-hydroxy estradiol; 4,17-dibenzoyl-4-hydroxy estradiol; 4,17-diacetyl-4-hydroxy estradiol; 3,4-diacetyl-4-hydroxy estradiol; 3,4,17-tribenzoyl-4-hydroxy estradiol; 3,4,17-triacetyl-4-hydroxy estradiol.

15. The composition of claim 1, wherein said estradiol metabolite is in a eutectic mixture.

16. The composition of claim 1, further comprising a hydrophilic polymer.

17. The composition of claim 16, wherein said hydrophilic polymer is selected from the group consisting of poly(ethylene glycol), poly(propylene glycol) and copolymers of poly(ethylene glycol) and poly(propylene glycol).

18. The composition of claim 1, wherein said estradiol metabolite is derivatized.

19. The composition of claim 18, wherein said derivative is selected from the group consisting of dicarboxylic acid compounds, diacids, polar compounds and ionic compounds.

20. The composition of claim 19, wherein such dicarboxylic acid compound is selected from the group consisting of oxalic, malonic, maleic, succinic, glutaric, adipic, pimelic and pamoic acid.

21. The composition of claim 19, wherein such diacids are selected from the group consisting of succinic, glutaric, maleic, malonic and oxalic acid.

22. The composition of any of claims 1-21, wherein said composition is produced by spray drying a solution of polymer and estradiol metabolite dissolved in an organic solvent.

23. The composition of any of claims 1-21, wherein said composition is produced by wet emulsification including a continuous and discontinuous phase followed by solvent removal.

24. The composition of claim 23, wherein said discontinuous phase contains estradiol metabolites and polymer.

25. The composition of claim 24, further comprising an additive.

26. The composition of claim 25, wherein said additive is selected from the group consisting of an antioxidant, a buffer and a release modifier.

27. The composition of claim 23, wherein said discontinuous phase contains an organic solvent.

28. The composition of claim 23, wherein said continuous phase further comprises an emulsifier.

29. The composition of claim 28, wherein said emulsifier is selected from the group consisting of phospholipids, lecithin, ionic surfactants, nonionic surfactants, poloxamers, polymers, polyvinyl pyrrolidone and polyvinyl alcohol.

30. The composition of claim 29, wherein said emulsifier is polyvinyl alcohol.

31. The composition of any of claims 1-21 wherein said composition was produced by the selective extraction of an oil phase solvent.

32. The composition of any of claims 1-21 wherein said composition was produced by an emulsion process.

33. The composition of any of claims 1-32, wherein said composition is produced by a process that includes an annealing step.

34. The composition of any of claims 1-33, wherein the composition is useful to treat an individual.

35. A treatment method comprising:
(a) administering to an individual a sustained release formulation containing an estradiol metabolite.

36. The method of claim 35, wherein said sustained release formulation containing an estradiol metabolite is selected from the group consisting of microparticles, nanoparticles, patches, crystals, gels, rods, stints, pellets, discs, lozenges, wafers, capsules, films, microcapsules, nanocapsules, hydrogels, liposomes, implants and vaginal rings.

37. The method of claim 36, wherein said microparticles or nanoparticles are comprised of a biodegradable polymer selected from the group consisting poly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoestex, biodegradable polyurethanes, blends and copolymers thereof.

38. The method of claim 37, wherein said microparticles or nanoparticles are comprised of poly(d,1-lactide-co-glycolide).

39. The method of claim 37, wherein said microparticles have a diameter between 1 and 200 micrometers.

40. The method of claim 37, wherein said nanoparticles have a diameter between 20 and 2000 nanometers.

41. The method of claim 37, wherein said microparticles or nanoparticles further comprise an additive.

42. The method of claim 41, wherein said additive is selected from the group consisting of butylated hydroxytoluene, propyl gallate, a tocopherol, ascorbyl palmitate, an antioxidant, a release modifier and a buffer.

43. The method of claim 35, wherein said estradiol metabolite is selected from the group consisting of 2-methoxy estradiol, 2-hydroxy estradiol, 4-methoxy estradiol and 4-hydroxy estradiol.

44. The method of claim 35, wherein said estradiol metabolite is delivered transdermally.

45. The method of claim 44, wherein said transdermal delivery is selected from the group consisting of buccal, oral, ocular, nasal, rectal and vaginal.

46. The method of claim 35, wherein said estradiol metabolite is a prodrug.

47. The method of claim 46, wherein said prodrug is an ester.

48. The method of claim 47, wherein said ester is selected from the group consisting of 3-benzoyl-2-methoxy estradiol; 17-benzoyl-2-methoxy estradiol; 17-acetyl-2-methoxy estradiol; 3-acetyl-2-methoxy estradiol; 3,17-dibenzoyl-2-methoxy estradiol; 3,17-diacetyl-2-methoxy estradiol; 3-benzoyl-4-methoxy estradiol; 17-benzoyl-4-methoxy estradiol; 17-acetyl-4-methoxy estradiol; 3-acetyl-4-methoxy estradiol; 3,17-dibenzoyl-4-methoxy estradiol; 3,17-diacetyl-4-methoxy estradiol; 3-benzoyl -2-hydroxy estradiol; 17-benzoyl-2-hydroxy estradiol; 17-acetyl-2-hydroxy estradiol; 3-acetyl-2-hydroxy estradiol; 3,17-dibenzoyl-2-hydmxy estradiol; 3,17-diacetyl-2-hydroxy estradiol; 2,3-dibenzoyl-2-hydroxy estradiol; 2,17-dibenzoyl-2-hydroxy estradiol; 2,17-diacetyl-2-hydroxy estradiol; 2,3-diacetyl-2-hydroxy estradiol; 2,3,17-tribenzoyl-2-hydroxy estradiol; 2,3,17-triacetyl-2-hydroxy estradiol; 3-benzoyl -4-hydroxy estradiol-, 17-benzoyl-4-hydroxy estradiol; 17-acetyl-4-hydroxy estradiol; 3-acetyl-4-hydroxy estradiol; 3,17-dibenzoyl-4-hydroxy estradiol; 3,17-diacetyl-4-hydroxy estradiol; 3,4-dibenzoyl-4-hydroxy estradiol; 4,17-dibenzoyl-4-hydroxy estradiol; 4,17-diacetyl-4-hydroxy estradiol; 3,4-diacotyl-4-hydroxy estradiol; 3,4,17-tribenzoyl-4-hydroxy estradiol; 3,4,17-triacetyl-4-hydroxy estradiol.

49. The method of claim 35, wherein said sustained release formulation containing an estradiol metabolite is in a eutectic mixture.

50. The method of claim 35, further comprising a hydrophilic polymer.

51. The method of claim 50, wherein said hydrophilic polymer is selected from the group consisting of poly(ethylene glycol), poly(propylene glycol) and copolymers of poly(ethylene glycol) and poly(propylene glycol).

52. The method of claim 35, wherein said estradiol metabolite is derivatized.

53. The method of claim 52, wherein said derivative is selected from the group consisting of dicarboxylic acid compounds, diacids, polar compounds and ionic compounds.

54. The method of claim 53, wherein such dicarboxylic acid compound is selected from the group consisting of oxalic, malonic, maleic, succinic, glutaric, adipic, pimelic and pamoic acid.

55. The method of claim 53, wherein such diacids is selected from the group consisting of succinic, glutaric, maleic, malonic and oxalic acid.

56. The method of any of claims 35-55, wherein said sustained release formulation containing an estradiol metabolite is produced by spray drying a solution of polymer and estradiol metabolite dissolved in an organic solvent.

57. The method of any of claims 35-55, wherein said sustained release formulation containing an estradiol metabolite is produced by wet emulsification including a continuous and discontinuous phase followed by solvent removal.

58. The method of claim 57, wherein said discontinuous phase contains estradiol metabolites and polymer.

59. The method of claim 58; further comprising an additive.

60. The method of claim 59, wherein said additive is selected from the group consisting of an antioxidant, a buffer, and a release modifier.

61. The method of claim 57, wherein said discontinuous phase contains an organic solvent.

62. The method of claim 61, wherein said organic solvent is selected from the group consisting of one solvent, two solvents and a mixture of solvents.

63. The method of claim 57, wherein said continuous phase further comprises an emulsifier.

64. The method of claim 63, wherein said emulsifier is selected from the group consisting of phospholipids, lecithin, ionic surfactants, nonionic surfactants, poloxamers, polymers, polyvinyl pyrrolidone and polyvinyl alcohol.

65. The method of claim 64, wherein said emulsifier is polyvinyl alcohol.

66. The method of any of claims 35-55, wherein said sustained release formulation containing an estradiol metabolite was produced by the selective extraction of an oil phase solvent.

67. The method of any of claims 35-55, wherein said sustained release formulation containing an estradiol metabolite was produced by an emulsion process.

68. A composition of matter comprising;
(a) an estradiol metabolite selected from the group consisting of catecholestrogens and methoxyestradiols; and
(b) a material providing for sustained release.

69. The composition of claim 68, wherein said material providing for sustained release is a microparticle or nanoparticle comprised of a biodegradable polymer selected from the group consisting of poly(lactide)s, poly(glycolide)s, poly(lactide-co-glycolide)s, poly(lactic acid)s, poly(glycolic acid)s, poly(lactic acid-co-glycolic acid)s, polycaprolactone, polycarbonates, polyesteramides, polyanhydrides, poly(amino acids), polyorthoesters, polyacetyls, polycyanoacrylates, polyetheresters, poly(dioxanone)s, poly(alkylene alkylate)s, copolymers of polyethylene glycol and polyorthoester, biodegradable polyurethanes, blends and copolymers thereof.

70. The composition of claim 68, wherein said estradiol metabolite is selected from the group consisting of 2-methoxy estradiol, 2-hydroxy estradiol, 4-methoxy estradiol and 4-hydroxy estradiol.
